# EUROPEAN PATENT APPLICATION

(11) **EP 3 954 758 A1**
(43) Date of publication of application: **16.02.2022**
(21) Application number: 20788404.0
(22) Date of filing: 09.04.2020
(51) Int. Cl.: C12N 5/071, A61K 35/22, A61P 13/12

(54) **METHOD FOR INDUCING DIRECT REPROGRAMMING OF URINE CELL INTO RENAL PROGENITOR CELL AND PHARMACEUTICAL COMPOSITION COMPRISING RENAL PROGENITOR CELL REPROGRAMMED BY SAME METHOD FOR PREVENTING OR TREATING RENAL CELL INJURY DISEASE**

(30) Priority: 09.04.2019 KR 20190041289
(71) Applicant: Korea University Research and Business Foundation, Seoul 02841 (KR)
(72) Inventor: YOU, Seung Kwon, Yongin-si Gyeonggi-do 17163 (KR); KIM, In Yong, Seoul 02475 (KR); GAO, Wei Wei, Seoul 02841 (KR); KANG, Phil Jun, Anyang-si Gyeonggi-do 13943 (KR); YUN, Won-Jin, Yongin-si Gyeonggi-do 17103 (KR); PARK, Gyuman, Seongnam-si Gyeonggi-do 13441 (KR)
(74) Representative: Schiweck Weinzierl Koch Patentanwälte Partnerschaft mbB
(86) International application number: PCT/KR2020/004835
(87) International publication number: WO 2020/209636

(57) **Abstract**

The present invention relates to a method for inducing direct reprogramming of urine cells into renal progenitor cells and a pharmaceutical composition comprising the renal progenitor cells reprogrammed by the method for preventing or treating renal cell injury disease. The present invention can make the mass production of customized reprogrammed renal progenitor cells by using urine cells, which are somatic cells easily and repeatedly obtainable without inconvenience and pain and as such, can be applied to incurable disease fields expandable to the renal injury therapy and kidney regeneration fields and to the production of cell therapy products.

<110>    Korea University Research and Business Foundation

<120>    Method for Direct Conversion from Human Urine Cells into Nephron
         Progenitor Cells and Composition for Preventing or Treating
         Kidney Failure Comprising Induced Nephron Progenitor Cells
         Thereof

<130>    PP-B2407

<160>    22

<170>    KoPatentIn 3.0

<210>    1
<211>    21
<212>    DNA
<213>    Artificial Sequence

<220>
<223>    SIX2-f


<400>    1
ctcaaggcac actacatcga g                                                  21


<210>    2
<211>    21
<212>    DNA
<213>    Artificial Sequence

<220>
<223>    SIX2-r


<400>    2
gttgtggctg ttagaattgg a                                                  21


<210>    3
<211>    22
<212>    DNA
<213>    Artificial Sequence

<220>
<223>    CITED1-f


<400>    3
cagcatcact tcccgccaat tt                                                 22


<210>    4
<211>    21
<212>    DNA
<213>    Artificial Sequence

<220>
<223>    CITED1-r


<400>    4
ttgcgatctt tcaccgcaag g                                                  21

<210>	5
<211>	22
<212>	DNA
<213>	Artificial Sequence

<220>
<223>	WT1-f


<400>	5
tgtgtgctta cccaggctgc aa	22


<210>	6
<211>	22
<212>	DNA
<213>	Artificial Sequence

<220>
<223>	WT1-r


<400>	6
ccgggagaac tttcgctgac aa	22


<210>	7
<211>	22
<212>	DNA
<213>	Artificial Sequence

<220>
<223>	NCAM1-f


<400>	7
cgatctcatg gtttcgggat gg	22


<210>	8
<211>	22
<212>	DNA
<213>	Artificial Sequence

<220>
<223>	NCAM1-r


<400>	8
tcatcaaact gcacctgggc tg	22


<210>	9
<211>	20
<212>	DNA
<213>	Artificial Sequence

<220>
<223>	NANOG-f

<400>    9
atagcaatgg tgtgacgcag                                                    20


<210>    10
<211>    20
<212>    DNA
<213>    Artificial Sequence

<220>
<223>    NANOG-r


<400>    10
gattgttcca ggattgggtg                                                    20


<210>    11
<211>    24
<212>    DNA
<213>    Artificial Sequence

<220>
<223>    OCT4-f


<400>    11
gacaggggga ggggaggagc tagg                                               24


<210>    12
<211>    26
<212>    DNA
<213>    Artificial Sequence

<220>
<223>    OCT4-r


<400>    12
cttccctcca accagttgcc ccaaac                                             26


<210>    13
<211>    22
<212>    DNA
<213>    Artificial Sequence

<220>
<223>    Nephrin-f


<400>    13
tggctcggac caaaccaaca tt                                                 22


<210>    14
<211>    22
<212>    DNA
<213>    Artificial Sequence

<220>
<223>    Nephrin-r

<400>    14
agggcctcat acctgatgca ga                                              22


<210>    15
<211>    22
<212>    DNA
<213>    Artificial Sequence

<220>
<223>    Synaptopodocin-f


<400>    15
cgctcaccac accaacttct aa                                              22


<210>    16
<211>    22
<212>    DNA
<213>    Artificial Sequence

<220>
<223>    Synaptopodocin-r


<400>    16
ctagaaagtg gcaggctctg tg                                              22


<210>    17
<211>    20
<212>    DNA
<213>    Artificial Sequence

<220>
<223>    podocalyxin-f


<400>    17
cttgagacac agacacagag                                                 20


<210>    18
<211>    18
<212>    DNA
<213>    Artificial Sequence

<220>
<223>    podocalyxin-r


<400>    18
ccgtatgccg cacttatc                                                   18


<210>    19
<211>    21
<212>    DNA
<213>    Artificial Sequence

<220>
<223>    CD13-f

<400>    19
ccatgaaggc cgagttcaac a                                                21

<210>    20
<211>    21
<212>    DNA
<213>    Artificial Sequence

<220>
<223>    CD13-r

<400>    20
atgaaggcca gcaagtacgt g                                                21

<210>    21
<211>    22
<212>    DNA
<213>    Artificial Sequence

<220>
<223>    AQP1-f

<400>    21
atgccgacga catcaactcc ag                                               22

<210>    22
<211>    22
<212>    DNA
<213>    Artificial Sequence

<220>
<223>    AQP1-r

<400>    22
tgagtcggtg agcaactttg gg                                               22

## Description

### Technical Field

The present invention relates to a method of inducing direct reprogramming of urine cells into renal progenitor cells and a pharmaceutical composition for preventing or treating a renal cell injury disease, which includes renal progenitor cells reprogrammed by the above-mentioned method.

### Background of the Invention

Stem cells refer to cells having unlimited potential towards self-renewal or differentiation related to specific cells and tissues required in the body. Stem cells are classified into three types: embryonic stem cells (ES cells) isolated from early embryos, embryonic germ cells (EG cells) isolated from embryonic primordial germ cells, and multipotent adult progenitor cells (MAPC cells) isolated from adult tissues.

Since stem cells have the potential to develop into cells with specialized functions, they are being studied as a cell therapy for functional recovery and tissue regeneration of various organs, and recently, the scope of their application is expanding to include plastic surgery and aesthetics.

The role of adult stem cells in vivo may be roughly summarized in two ways. First, stem cells themselves are differentiated into tissues and cells in our bodies and regenerate damaged tissues and cells, and second, stem cells continuously secrete growth factors and proteins such as cytokines during its lifespan to aid the growth and regeneration of neighboring cells.

Meanwhile, a direct reprogramming method is a technique targeting somatic cells, which can be converted to produce cells with a different type of desired intrinsic function. Unlike the reprogramming-induced pluripotent stem cells (iPSCs) discovered by Professor Yamanaka (Japan), since not going through a pluripotency state, direct reprogramming has advantages in terms of karyotypic stability, homogeneity of target cells, variability between cell sources, the risk of tumorigenesis, the specificity according to a patient, and the efficiency of time and effort. However, the current direct reprogramming method mainly uses mouse cells as a cell source so that the probability of being equally reproducible in human somatic cells is not high. Among human somatic cells, skin cells (fibroblasts) are often used, but in this case, an invasive method of collecting cell sources is needed, thereby causing pain and a safety risk for a donor, and decreasing convenience.

While the presence of renal progenitor cells in the kidney, which has a very limited capacity for spontaneous regeneration after injury, remains a question until now, the mechanism of renal regeneration in academic circles is as follows. Surviving renal cells after injury may be temporarily dedifferentiated and grow, which is based on the critical role of renal progenitor cells that can replace injured tissue (Benigni A et al., Lancet 375(9722): 1310-7, 2010; Sallustio F et al., Biores Open Access 4(1):326-33, 2015). That is, in the regeneration process, progenitor cells proliferated from renal progenitor cells migrate to the injured area and are reconstituted into functional kidney tissue through growth and differentiation. In addition, in the regeneration process, through autocrine, paracrine and endocrine interactions by stem cells, it contributes to physiological cell transformation of the kidney and regeneration of each renal compartment (Bussolati B et al., Am J Pathol. 66(2):545-55, 2005; Sagrinati C et al., J Am Soc Nephrol. 17(9):2443-56, 2006).

Therapeutic approaches using progenitor cells contribute to reducing inflammatory responses. After renal injury, ROS stress caused by inflammatory factors and related endovascular effectors is considered as another barrier of tissue regeneration. This does not only expand renal injury, but also has a risk of causing a cardiovascular disease in patients with renal failure.

In terms of renal injury, the production of renal progenitor cells induced from somatic cells, and the technology of developing pharmaceutical materials using the same have high value for renal tissue treatment and regeneration in terms of efficiency, patient-specific treatment and therapeutic efficacy, and it is meaningful in that they present a new bio-industry model along with pioneering a new market (Takasato M et al., Semin Nephrol. 4(4):462-80, 2014; Sallustio F et al., Biores Open Access. 4(1):326-33, 2015).

Since renal progenitor cells does not only replace cells, but also secrete therapeutic factors playing an important role in renal regeneration, the use of kidney-specific renal progenitor cells for developing an agent for preventing and regeneratively treating renal injury is a very natural scientific approach even in terms of an expected therapeutic effect.

Therefore, the inventors had made an earnest effort to prepare renal progenitor cells that can regenerate or replace injured renal tissue, and thus induced reprogrammed renal progenitor cells having similar characteristics into renal progenitor cells by culturing overexpressed urine cells by introducing reprogramming factors such as Oct4, Sox2, Klf4, c-Myc and Slug in an environment (niche) including factors considered important in renal development, and confirmed tissue regeneration efficacy using the reprogrammed renal progenitor cells, and thus the present invention was completed.

### Technical Problem

The present invention is directed to providing a method of inducing direct reprogramming from urine cells to renal progenitor cells, which includes introducing reprogramming factors such as Oct4, Sox2, Klf4, c-Myc and Slug into urine cells, and culturing the cells in an environment (niche) including factors considered important in renal development to induce reprogrammed renal progenitor cells having similar characteristics into renal progenitor cells applicable to renal injury healing, renal regeneration and treatment of a renal cell injury disease.

### Summary of the Invention

To achieve the purpose, the present invention provides a method of inducing direct reprogramming from urine cells to renal progenitor cells, which includes: (a) culturing urine cells isolated from urine; (b) introducing reprogramming factors such as i) a nucleic acid encoding an Oct4 protein, ii) a nucleic acid encoding an Sox2 protein, iii) a nucleic acid encoding an Klf4 protein, and iv) a nucleic acid encoding an c-Myc protein and v) a nucleic acid encoding a Slug protein into the cultured urine cells; (c) culturing the reprogramming factor-introduced urine cells in a renal progenitor cell culture medium to induce reprogramming into renal progenitor cells; and (d) selecting the reprogrammed renal progenitor cells having the characteristics of renal progenitor cells from the cells in which direct reprogramming into renal progenitor cells is induced.

The present invention also provides a composition for preventing or treating a renal cell injury disease, which includes reprogrammed renal progenitor cells as an active ingredient.

The present invention also provides a method of preventing or treating a renal cell injury disease, which includes administering a composition containing renal progenitor cells reprogrammed by the above-described method as an active ingredient into a subject.

The present invention also provides a use of a composition containing renal progenitor cells reprogrammed by the above-described method as an active ingredient for preventing or treating a renal cell injury disease.

The present invention also provides a use of a composition containing renal progenitor cells reprogrammed by the above-described method as an active ingredient for preparing a drug for preventing or treating a renal cell injury disease.

### Description of Drawings

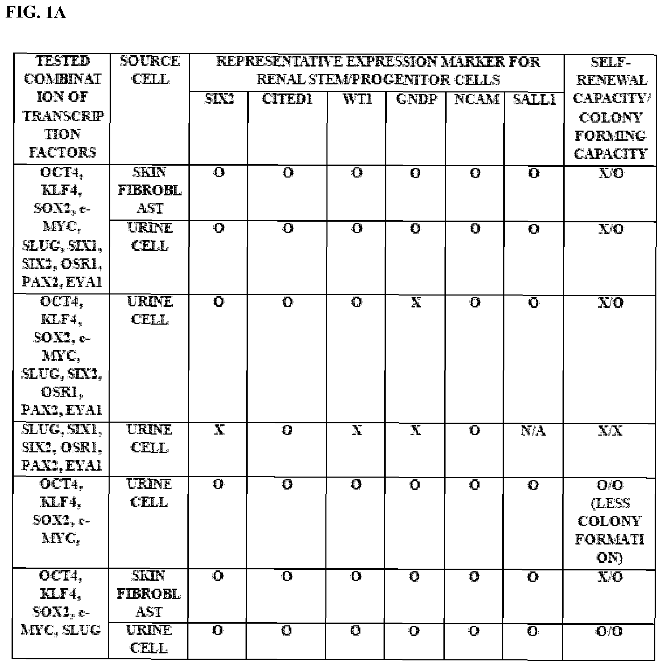

**FIG. 1A** shows the combination of transcription factors used to try to induce reprogramming into renal progenitor cells using direct reprogramming technology, and the colony forming capacity and self-renewal capacity of cells induced thereby.

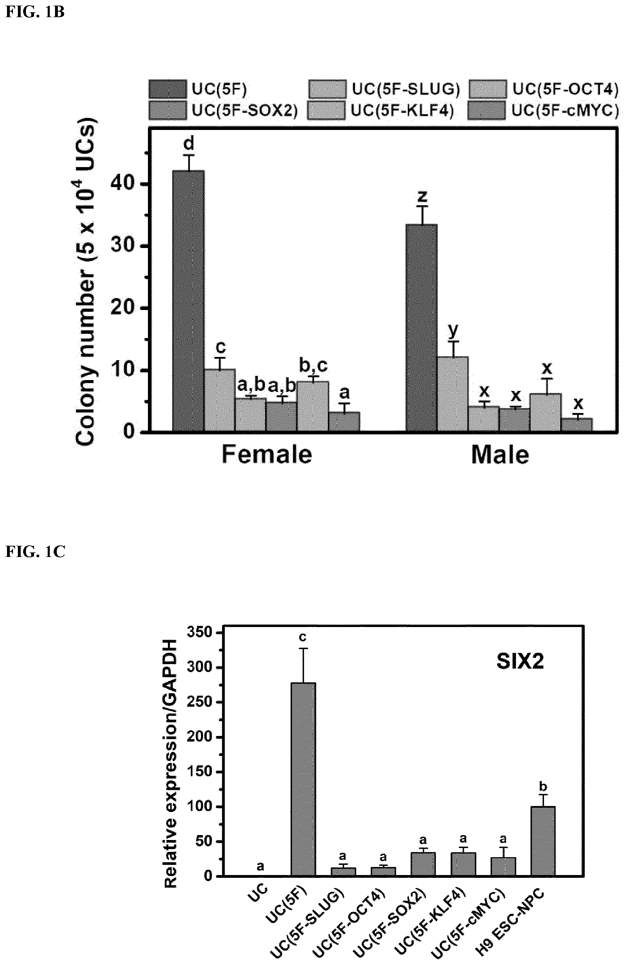

**FIG. 1B** shows the result of comparing the colony forming capacity of female and male-derived urine cells induced according to the combination of Oct4, Sox2, Klf4, c-Myc and Slug transcription factors (5F: the combination of Oct4, Sox2, Klf4, c-Myc and Slug; 5F-Slug : the combination of Oct4, Sox2, Klf4 and c-Myc).
**FIG. 1C** shows the result of comparing expression levels of SIX2, which is a marker gene for renal progenitor cells, according to the combination of Oct4, Sox2, Klf4, c-Myc and Slug.

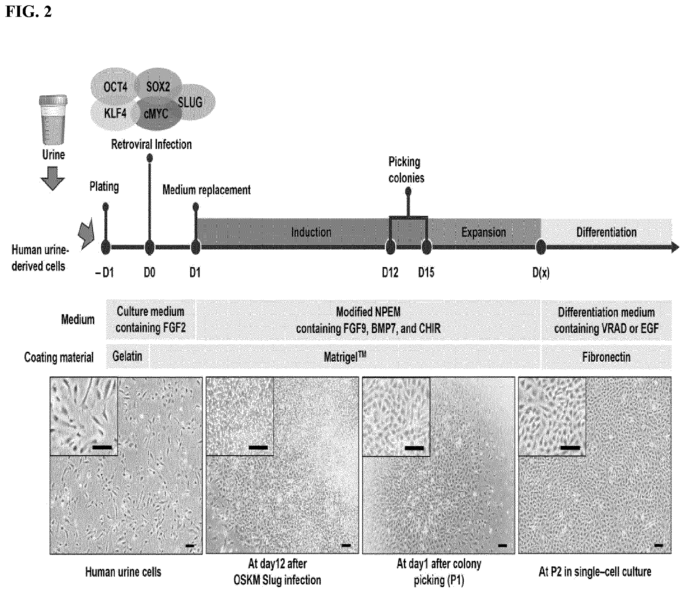

**FIG. 2** illustrates the entire process in which a colony is obtained by inducing urine cells into renal progenitor cells using direct reprogramming technology, the number of the renal progenitor cells expands through corresponding colony culture, and then the renal progenitor cells are differentiated into renal cells.

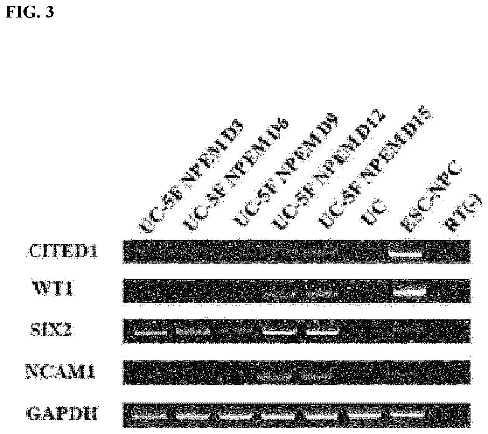

**FIG. 3** shows the result of confirming whether renal progenitor cell marker genes such as SIX2, CITED1 and WT1 are expressed through analysis of mRNA levels by RT-PCR using renal progenitor cells derived from embryonic stem cells as a positive control after reprogrammed renal progenitor cells are induced from urine cells by introducing OCT4, SOX2, KLF4, cMYC and SLUG genes.

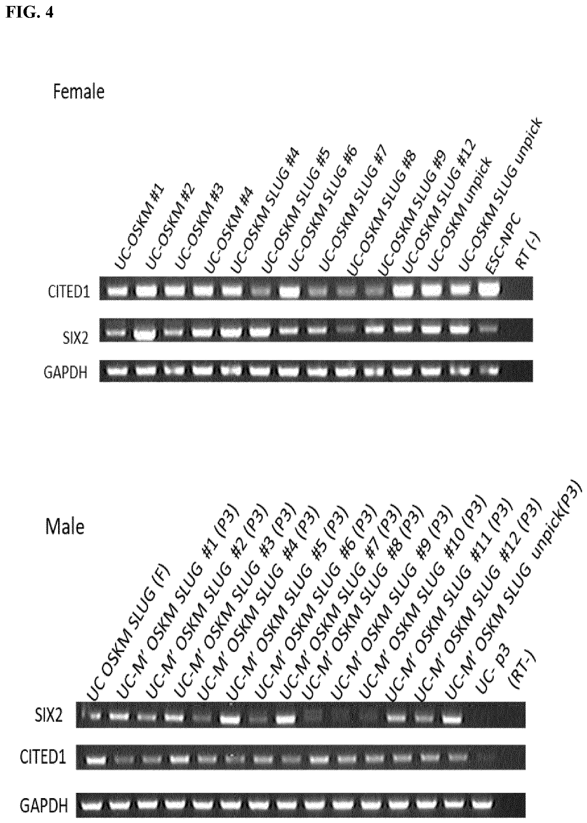

**FIG. 4** shows the result of confirming whether renal progenitor cell marker genes such as SIX2, CITED1 and WT1 are expressed through the analysis of mRNA levels by RT-PCR by expansion culture after a colony in which female or male-derived urine cells are induced into reprogrammed renal progenitor cells is selected.

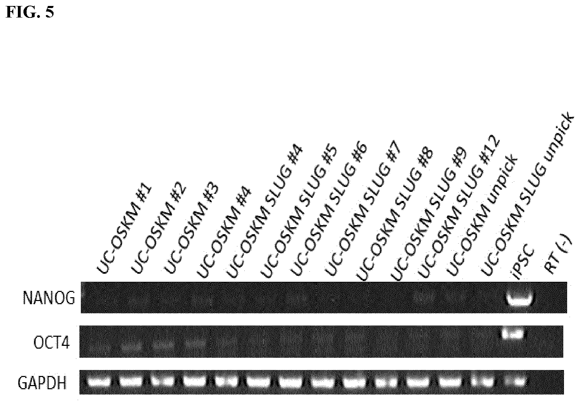

**FIG. 5** shows the result of confirming stability from the risk of tumorigenesis, which pluripotent stem cells have, by confirming whether pluripotent marker genes such as NANOG and OCT4 are expressed through the analysis of mRNA levels by RT-PCR, after reprogrammed renal progenitor cells are induced using urine cells.

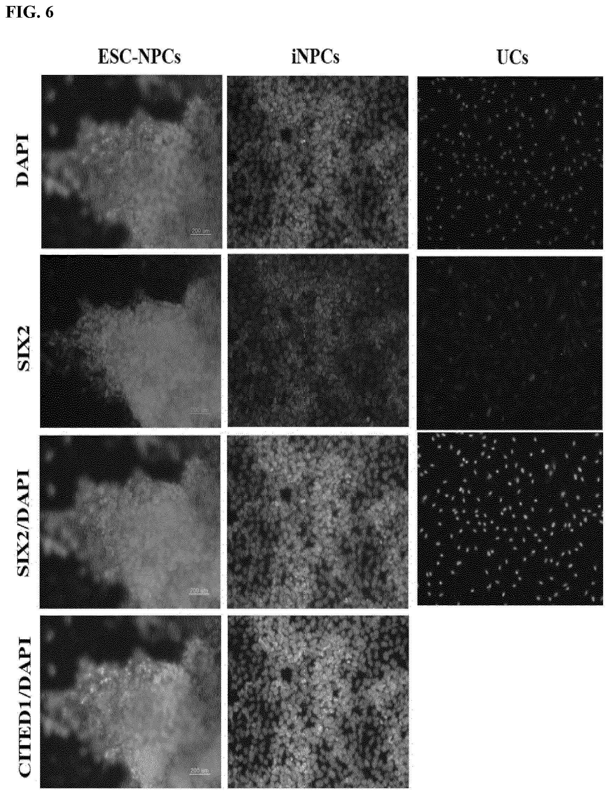

**FIG. 6** shows the result of confirming whether reprogrammed renal progenitor cells express renal progenitor cell marker genes such as SIX2 and CITED1 through immunoassay.

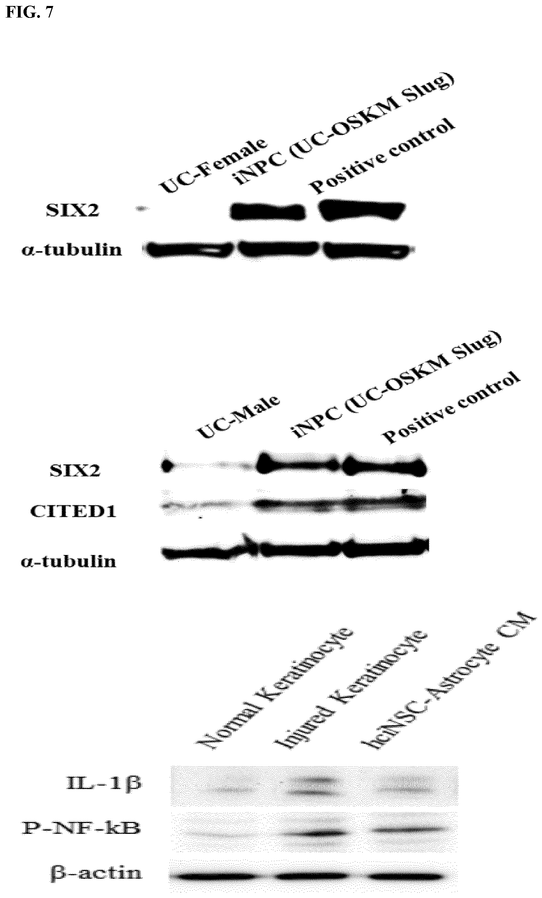

**FIG. 7** shows the western blotting result of confirming whether reprogrammed renal progenitor cells express renal progenitor cell marker proteins such as SIX2 and CITED1 using female or male-derived urine cells.

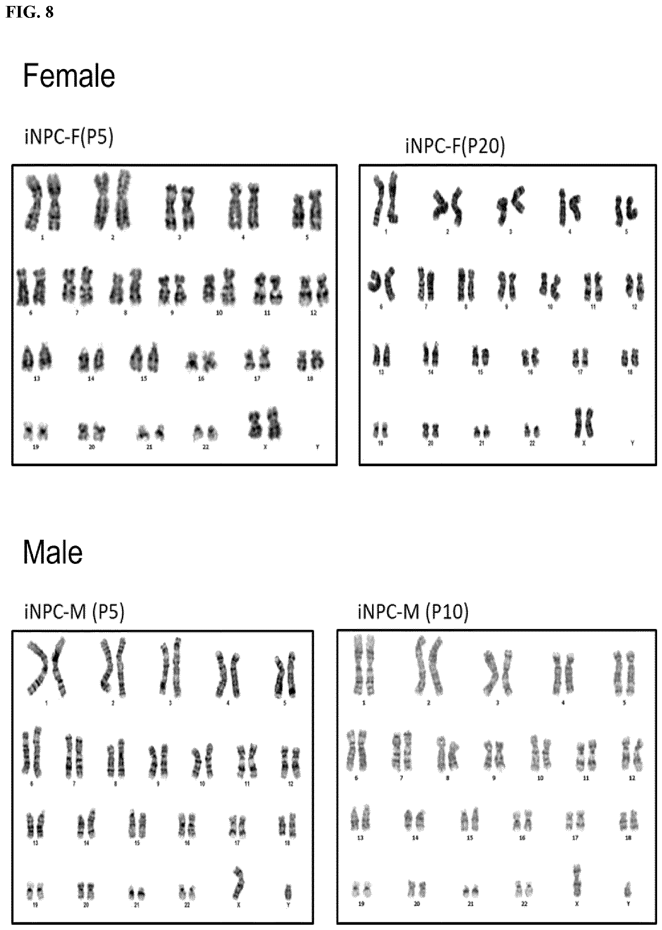

**FIG. 8** shows the karyotype analysis result according to time confirming whether reprogrammed renal progenitor cells conserve a normal chromosome using female or male-derived urine cells.

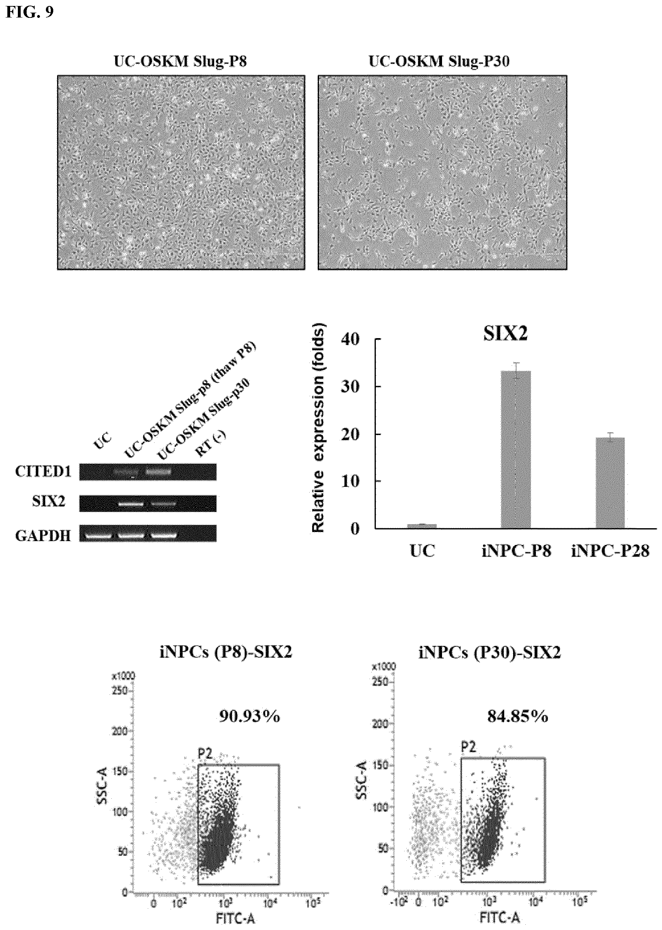

**FIG. 9** shows the mRNA level analysis and FACS results of confirming whether, in reprogrammed renal progenitor cells, the expression of renal progenitor cell marker genes such as SIX2 and CITED 1 is maintained in a renal progenitor cell expansion culture medium for a long period of time.

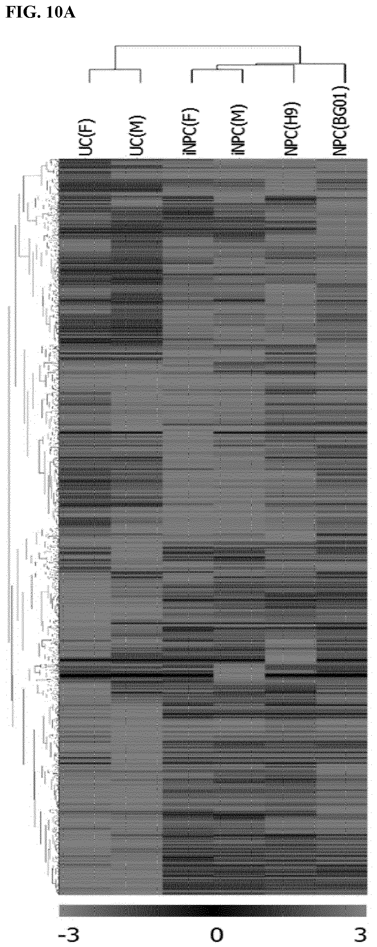

**FIGS. 10A to 10D** show the results of confirming whether reprogrammed renal progenitor cells show mRNA and long non-coding-RNA (lnc-RNA) expression patterns, which are similar to renal progenitor cells derived from H9 and BG01 embryonic stem cells rather than original female or male urine-derived cells at a global gene expression level through total RNA sequencing.

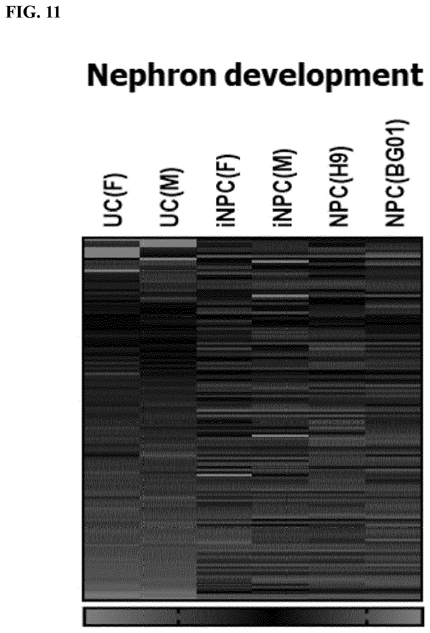

**FIG. 11** shows the result of confirming whether reprogrammed renal progenitor cells show mRNA and lnc-RNA expression patterns, which are similar to renal progenitor cells derived from H9 and BG01 embryonic stem cells rather than original female or male urine-derived cells at an expression level of a renal development-related gene among all genes.

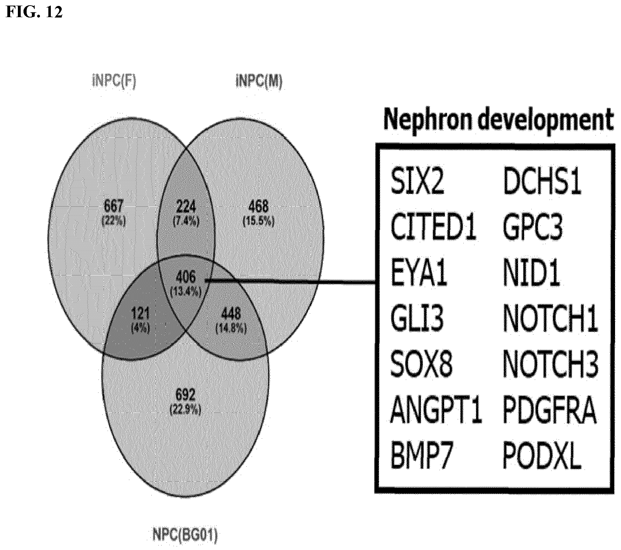

**FIG. 12** shows the Venn diagram of the renal development-related mRNA expression commonality between renal progenitor cells reprogrammed from female or male urine cells and renal progenitor cells derived from BG01 embryonic stem cells at a global gene expression level through total RNA sequencing.

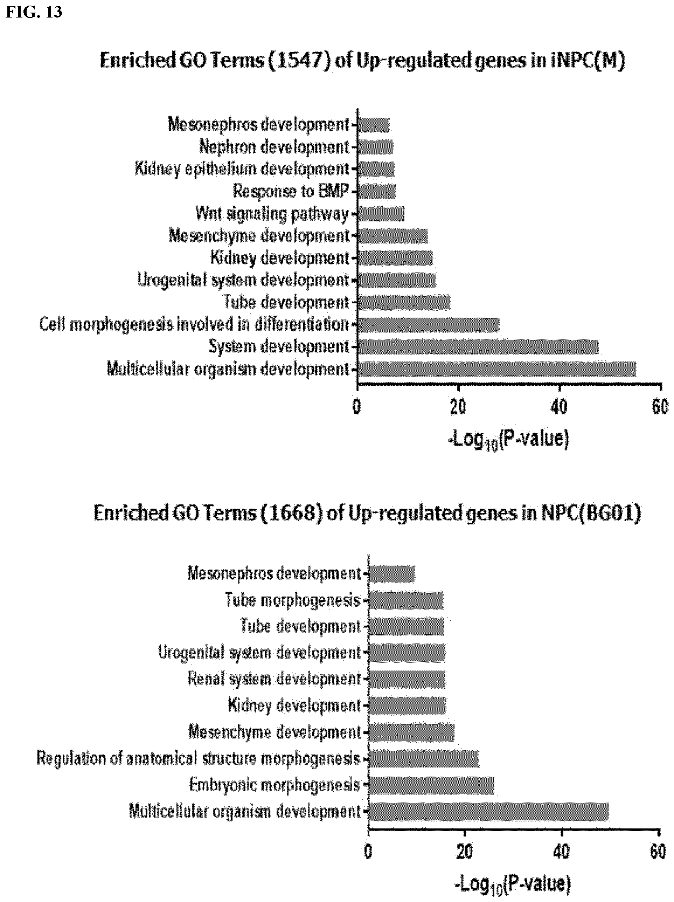

**FIG. 13** shows the result of comparing the similarity between renal progenitor cells induced from urine cells and renal progenitor cells derived from BG01 embryonic stem cells at an expression level of a renal development-associated gene of each group by being divided from all expressed genes by details through total RNA sequencing.

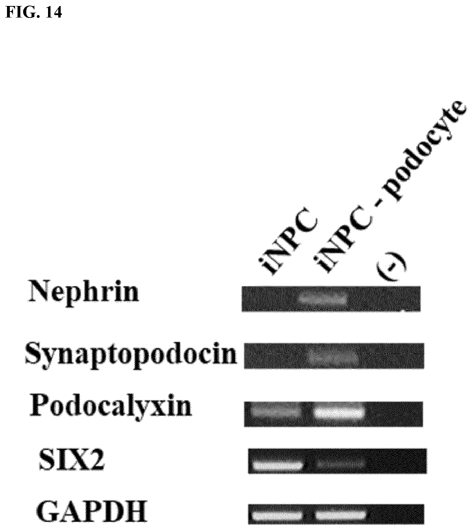

**FIG. 14** shows the result of confirming the expression of glomerular podocyte gene markers such as nephrin, synaptopodocin and podocalyxin through mRNA level analysis by RT-PCR to verify differentiation capacity of reprogrammed renal progenitor cells into glomerular podocytes.

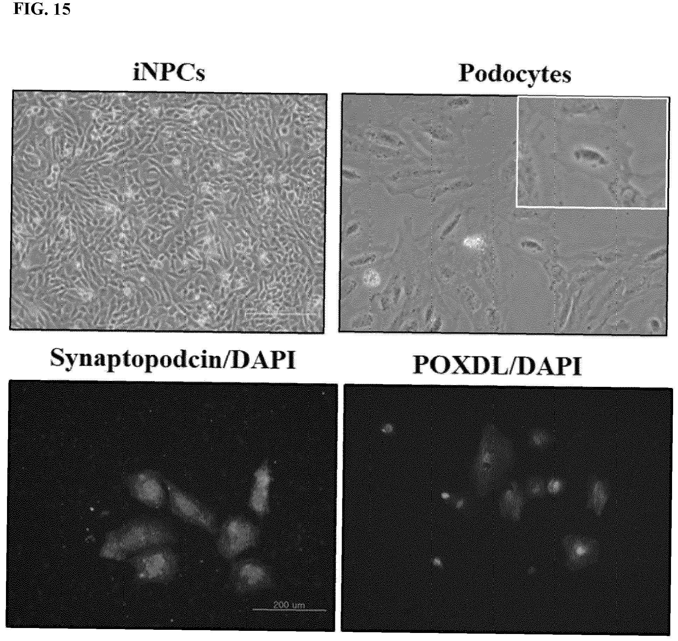

**FIG. 15** shows the results of comparing the specific shape of glomerular podocytes with a pre-differentiation shape thereof through microscopy and confirming the expression of glomerular podocyte gene markers such as synaptopodocin and POXDL through immunostaining to verify the differentiation capacity of reprogrammed renal progenitor cells into glomerular podocytes.

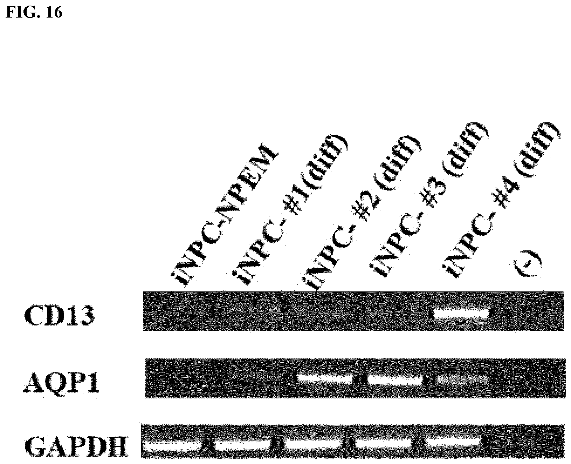

**FIG. 16** shows the result of confirming the expression of renal tubular cell gene markers such as CD13 and AQP1 through mRNA level analysis by RT-PCR to verify the differentiation capacity of reprogrammed renal progenitor cells into renal tubular cells.

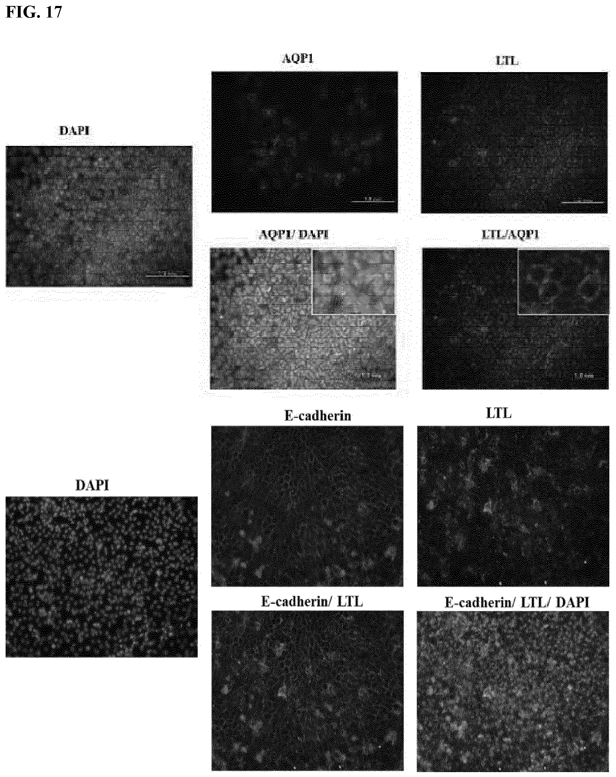

**FIG. 17** shows the result of confirming the expression of renal tubular cell gene markers such as AQP1 and LTL through immunostaining to verify the differentiation capacity of reprogrammed renal progenitor cells into renal tubular cells.

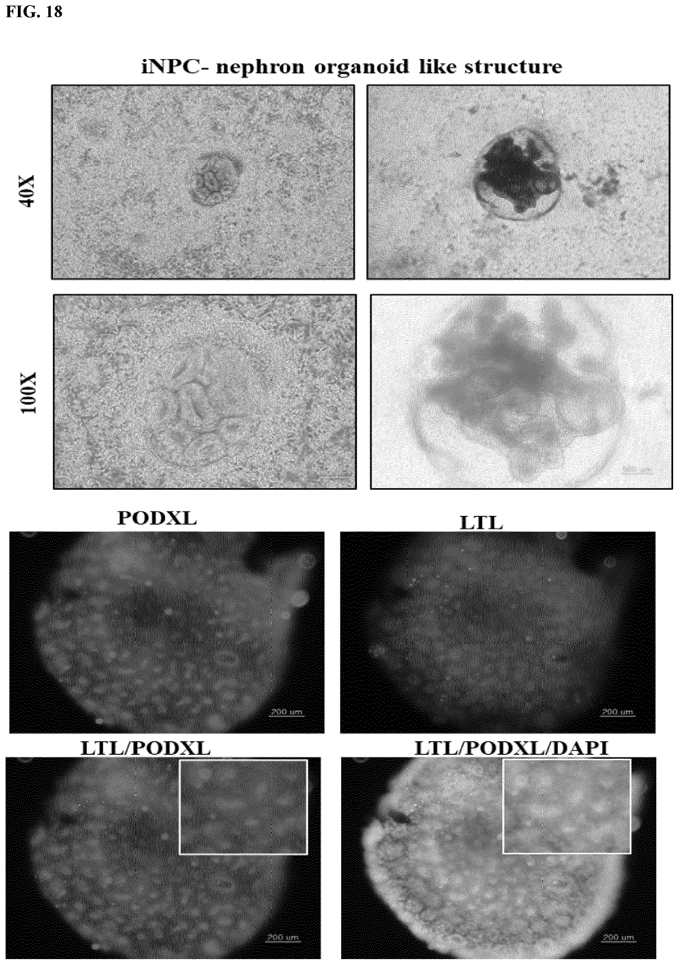

**FIG. 18** shows the results of confirming the specific shape of nephron-like tissue and the expression of glomerular podocyte gene marker such as PODXL and renal tubular cell gene marker such as LTL through immunostaining to verify the differentiation capacity of reprogrammed renal progenitor cells into nephron-like tissue.

### Detailed Description of the Invention

Unless defined otherwise, all technical and scientific terms used herein have the same meaning as commonly understood by one of ordinary skill in the art to which the present invention belongs. In general, the nomenclature used herein is well known and commonly used in the art.

In the present invention, urine cells were isolated from urine and cultured, and a vector into which the combination of Oct4, Klf4, Sox2, c-Myc and Slug reprogramming factors is introduced was introduced into the urine cells. The urine cells into which the reprogramming factors were introduced were cultured by adding FGF9, BMP7, CHIR99021 and Y-27632 to an Advanced RPMI 1640 medium containing heparin, LDN-193189 and L-glutamine, thereby inducing reprogramming into renal progenitor cells.

Accordingly, in one aspect, the present invention provides a method of inducing direct reprogramming from urine cells to renal progenitor cells, which includes: (a) culturing urine cells isolated from urine; (b) introducing reprogramming factors such as i) a nucleic acid encoding an Oct4 protein, ii) a nucleic acid encoding an Sox2 protein, iii) a nucleic acid encoding an Klf4 protein, and iv) a nucleic acid encoding an c-Myc protein and v) a nucleic acid encoding a Slug protein into the cultured urine cells; (c) culturing the reprogramming factor-introduced urine cells in a renal progenitor cell culture medium to induce reprogramming into renal progenitor cells; and (d) selecting the reprogrammed renal progenitor cells having the characteristics of renal progenitor cells from the cells in which direct reprogramming into renal progenitor cells is induced.

The term "reprogrammed renal progenitor cells" used herein refers to cells made in the manner of establishing undifferentiated stem cells having pluripotency which is similar or the same as renal stem cells from differentiated cells using reprogramming technology. The induced renal progenitor cells have characteristics the same or similar to renal progenitor cells, and specifically show similar cell morphology, and have similar gene and protein expression patterns and pluripotency in vivo and ex vivo. Accordingly, the reprogrammed renal progenitor cells of the present invention are capable of being differentiated into glomerular podocytes or renal tubular cells.

The term "renal progenitor cells" used herein are multipotent undifferentiated cells (stem cells and/or progenitor cells) which can be differentiated into renal tissue-constituting cells, and also include undifferentiated cells capable of being differentiated into podocytes and renal tubular cells.

The "reprogramming (dedifferentiation)" used herein refers to a phenomenon of acquiring a 'stem cell'-like or multipotent cell state before differentiation of differentiated cells into one or more different tissue types.

The term "differentiation" used herein refers to a phenomenon in which the structure or function of a cell is specialized during growth after cell division and proliferation. Multipotent mesenchymal stem cells may be further differentiated into a different type of progenitor cells after being differentiated into lineage-limited progenitor cells (e.g., mesodermal cells), and then differentiated into terminally differentiated cells playing a specific role in specific tissue (e.g., adipocytes, osteocytes, chondrocytes, etc.).

The "renal progenitor cells" used herein may be differentiated into terminally-differentiated cells, that is, kidney-constituting cells, which play a specific role in specific tissue (e.g., renal tissue).

The term "urine cells (UCs)" used herein can be easily and repeatedly acquired at any time, without any inconvenience and pain, safely, simply and at low cost, regardless of a patient's age, gender or health condition, and are known as somatic cells that can be obtained from urine without any special isolation process.

In the present invention, the urine cells may be urine-derived somatic cells.

In the present invention, when reprogramming factors such as Oct4, Sox2, Klf4 and c-Myc and a transcription factor Slug are expressed in urine cells, the possibility of reprogramming (de-differentiation) an already differentiated cell type, urine cells, into renal progenitor cells with differentiation capacity was found.

The term "reprogramming factor" used herein started from reprogramming, which is a concept introduced by Professor Yamanaka's team in 2006. All types of tissue of an adult are gradually differentiated from an undifferentiated state (not differentiated) throughout a normal development process, and changed into cells specialized for each function. Among them, cells of a fertilized egg are totipotent, and when becoming blastocysts as development progresses, can be divided into the inner cell mass and outer cells. At this time, the inner cell mass cells may develop into embryonic somatic cells and germ cells, which are called pluripotent. The embryonic stem cells show gene expression patterns unique to pluripotency, and representative examples thereof include Oct4, Sox2, Nanog, and Lin28. Reprogramming is the technology that induces the expression of a specific gene in somatic cells and restores properties similar to those of undifferentiated cells such as embryonic and adult stem cells. Oct4, Sox2, Klf4 and c-Myc are various factors that have been used in a series of studies thereof, and were used for reprogramming from urine cells to renal progenitor cells in the present invention

The "Slug" used herein is also known as "Snai2," and Slug and Snai2 can be understood as being the same.

The Oct4. Sox2, Klf4, c-Myc, and Slug (or Snai2) according to the present invention include all Oct4, Sox2, Klf4, c-Myc, Slug (or Snai2) derived from an animal such as a human, a horse, sheep, a pig, a goat, a camel, an antelope and a dog, and preferably, human Oct4, Sox2, Klf4, c-Myc and Slug (or Snai2). In addition, the Oct4, Sox2, Klf4, c-Myc, and Slug (or Snai2) proteins of the present invention used in reprogramming into renal progenitor cells may include not only proteins having a wild-type amino acid sequence, but also mutants of the Oct4, Sox2, Klf4, c-Myc and Slug (or Snai2) proteins.

In an exemplary embodiment, the sequences of Oct4, Sox2, Klf4, c-Myc, and Slug (or Snai2) genes used for reprogramming in the present invention were disclosed in Cell 2007 Nov 30; 131(5):861-72.

The mutants of the Oct4, Sox2, Klf4, c-Myc, and Slug (or Snai2) proteins refer to proteins having different sequences from the natural amino acid sequences of Oct4, Sox2, Klf4, c-Myc, and Slug (or Snai2) due to the deletion, insertion, non-conservative or conservative substitution of one or more amino acid residues or a combination thereof. The mutants may be functional equivalents that exhibit the same biological activity as natural proteins, or mutants whose physicochemical properties are modified as needed. The mutants are mutants with increased structural stability to physical and chemical environments or increased physiological activity.

A nucleic acid consisting of the nucleotide sequence encoding the Oct4, Sox2, Klf4, c-Myc, or Slug (or Snai2) protein is a nucleic acid consisting of the nucleotide sequence encoding the mutant-type Oct4, Sox2, Klf4, c-Myc, or Slug (or Snai2) protein as described above. The nucleic acid may be modified by substitution, deletion, insertion of one or more bases or a combination thereof, and may be isolated from nature or prepared using a chemical synthesis method.

The nucleic acid having the nucleotide sequence encoding the Oct4, Sox2, Klf4, c-Myc, or Slug (or Snai2) protein may be single-stranded or double-stranded, and may be a DNA molecule (genome or cDNA) or an RNA molecule.

In one preferable aspect, the reprogramming factors inducing reprogramming of urine cells into renal progenitor cells according to the present invention may include vectors expressing Oct4, Sox2, Klf4, c-Myc, and Slug (or Snai2) proteins into which nucleic acids having nucleotide sequences encoding Oct4, Sox2, Klf4, c-Myc, and Slug (or Snai2) proteins are introduced.

In the present invention, in Step (b), the reprogramming factor-inserted virus vector may be directly introduced into urine cells.

The term "vector" used herein refers to an expression vector capable of expressing a target protein in appropriate host cells, and a gene construct comprising essential regulatory elements operably linked such that a gene insert is expressed.

The term "operably linked" used herein refers to the functional linkage of a nucleic acid sequence encoding a desired protein with a nucleic acid expression regulatory sequence. The operable linkage with a recombinant vector may be formed using a gene recombination technique well known in the related art, and site-specific DNA cleavage and linkage use enzymes generally known in the related art.

The vector of the present invention may include a signal sequence or leader sequence for membrane targeting or secretion, in addition to expression regulatory elements such as a promoter, an operator, an initiation codon, a termination codon, a poly-adenylation signal and an enhancer, and may be prepared in various types depending on its purpose. The promoter of the vector may be a constitutive or inducible vector. In addition, the expression vector includes a selectable marker for selecting host cells containing a vector, and a replicable expression vector includes a replication origin. The vector may be self-replicating or integrated into host DNA.

The vector includes a plasmid vector, a cosmid vector and a virus vector. Preferably, the vector is a virus vector. The virus vector includes vectors derived from retroviruses, for example, human immunodeficiency virus (HIV), murine leukemia virus (MLV), avian sarcoma/leukosis (ASLV), spleen necrosis virus (SNV), Rous sarcoma virus (RSV) and mouse mammary tumor virus (MMTV), adenovirus, adeno-associated virus, and herpes simplex virus, but the present invention is not limited thereto. In an exemplary embodiment of the present invention, a pMXs vector was used as a murine Moloney leukemia virus (MMLV)-based virus vector.

In the present invention, the nucleic acids having nucleotide sequences encoding Oct4, Sox2, Klf4, c-Myc, and Slug (or Snai2) proteins may be delivered to cells by a known method in the art, for example, via vector-type naked DNA (Wolff et al. Science,1990: Wolffet al. J Cell Sci. 103:1249-59, 1992), or using a liposome or a cationic polymer. A liposome is a phospholipid membrane prepared by mixing cationic phospholipids such as DOTMA or DOTAP for gene delivery, and when a cationic liposome and an anionic nucleic acid are mixed in a predetermined ration, a nucleic acid-liposome complex is formed.

In still another aspect, the composition for inducing reprogramming of urine cells into renal progenitor cells may include a virus expressing Oct4, Sox2, Klf4, c-Myc and Slug (or Snai2) proteins including nucleic acids consisting of the nucleotide sequences encoding the Oct4, Sox2, Klf4, c-Myc, and Slug (or Snai2) proteins.

The term "virus" used herein refers to a virus expressing Oct4, Sox2, Klf4, c-Myc, and Slug (or Snai2) constructed by transformation and infection of packaging cells with a virus vector including the nucleic acids having the nucleotide sequences encoding Oct4, Sox2, Klf4, c-Myc and Slug (or Snai2) proteins.

The virus that can be used in preparation of a virus expressing -Oct4, Sox2, Klf4, c-Myc, and Slug (or Snai2) proteins of the present invention may be a retrovirus, an adenovirus, an adeno-associated virus or a herpes simplex virus, but the present invention is not limited thereto. Preferably, the virus is a retrovirus.

In one exemplary embodiment of the present invention, a virus expressing Oct4, Sox2, Klf4, c-Myc, and Slug (or Snai2) proteins was prepared by transforming packaging cells, 293GPG cells, which produce high-titer viruses capable of infection of a wide range of host cells with Oct4, Sox2, Klf4, c-Myc, and Slug (or Snai2), with vectors prepared by inserting nucleic acid sequences encoding Oct4, Sox2, Klf4, c-Myc, and Slug (or Snai2) proteins into pMXs vectors (pMXs-Oct4, pMXs-Sox2, pMXs-Klf4, pMXs-c-Myc, and pMXs-Slug (or pMXs-Snai2)) to infect urine cells.

In the present invention, in Step (a), the culture of urine cells may be performed in a fetal bovine serum (FBS)-, basic fibroblast growth factor (bFGF)- or epithelial growth factor (EGF)-containing medium.

The term "culture medium" used herein refers to a medium that can support the growth and survival of cells *in vitro,* and includes all of conventional media which are used in the art suitable for induction and culture of urine cells and reprogrammed renal progenitor cells. The medium and culture conditions may be selected according to the type of cells. The medium used for culture is preferably a cell culture minimum medium (CCMM), and generally contains a carbon source, a nitrogen source and trace elements. Such cell culture minimal medium contains, for example, Dulbecco's Modified Eagle's Medium (DMEM), Minimal essential Medium (MEM), Basal Medium Eagle (BME), RPMI1640, F-10, F-12, α-Minimal essential Medium (&MEM), Glasgow's Minimal essential Medium (GMEM), and Iscove's Modified Dulbecco's Medium (IMEM), but the present invention is not limited thereto. In addition, the medium may contain an antibiotic such as penicillin, streptomycin or gentamicin.

In an exemplary embodiment of the present invention, cells isolated from urine may be obtained by culturing in a basal medium containing FBS, bFGF and EGF, and specifically, may be cultured in an FBS-containing high glucose DMEM and Renal Epithelial Cell Growth Medium (REGM, Lonza) by adding bFGF and EGF. More preferably, the high glucose DMEM and REGM medium further contains L-glutamine and penicillin-streptomycin.

In the present invention, in Step (b), the method of introducing a reprogramming factor into urine cells may be any method of providing a nucleic acid molecule or protein to cells conventionally used in the art without limitation, and preferably, a method of administering a reprogramming factor into a cell culture or a method of directly injecting a reprogramming factor into differentiated cells. The reprogramming factor used herein may be a virus obtained from packaging cells transfected with a virus vector into which a gene of the corresponding factor is inserted, mRNA produced by *in vitro* transcription, or a protein produced in each of various cell lines. In one exemplary embodiment of the present invention, the introduction of the reprogramming factor into urine cells used DNA encoding Oct4, Sox2, Klf4, c-Myc, or Slug (or Snai2).

The method of directly injecting DNA into differentiated cells may be one suitably selected from the methods known in the art, for example, microinjection, electroporation, particle bombardment, direct muscle injection, and methods using an insulator and transposon, but is not limited thereto. Specifically, in one embodiment of the present invention, DNA of the reprogramming factor was introduced into urine cells using electroporation.

In the present invention, in Step (c), a medium inducing urine cells into which nucleic acids encoding Oct4, Sox2, Klf4, c-Myc, and Slug proteins are introduced into renal progenitor cells (renal progenitor cell-inducing medium) may induce reprogramming into renal progenitor cells by culturing in a basal medium containing one or more of LDN-193189, CHIR99021, FGF9 and BMP7, and in an exemplary embodiment of the present invention, it was confirmed that the medium has the highest conversion rate into renal progenitor cells in a medium prepared by adding all of Y-27632, CHIR99021, FGF9 and BMP7 to an Advanced RPMI 1640 medium and additionally adding LDN-193189, heparin and L-glutamine.

In the present invention, the culture medium in Step (c) preferably includes FGF9, BMP7, CHIR99021 and Y-27632, and more preferably further includes heparin, LDN-193189 or L-glutamine, and is included in an Advanced RPMI 1640 basal medium, but the present invention is not limited thereto.

In addition, the culture in the culture medium may have increased induction efficiency when performed under one or more coating conditions of Matrigel, laminin, fibronectin, gelatin and collagen.

In the present invention, the selection of the induced renal progenitor cells in Step (d) may be collecting a colony of renal progenitor cells produced after performing Step (c), and the selected renal progenitor cells may be cultured in a renal progenitor cell medium.

The renal progenitor cell medium may be a medium to which all of FGF9, BMP7, Y-27632, CHIR99021, heparin, LDN-193189 and L-glutamine are added to an Advanced RPMI 1640 medium. In addition, the reprogrammed renal progenitor cells may be subcultured using a 0.5 mM EDTA solution or Accutase solution.

In yet another aspect, the present invention provides a pharmaceutical composition for preventing or treating a renal cell injury disease, which includes renal progenitor cells reprogrammed by the above-described method as an active ingredient.

In yet another aspect, the present invention provides a method of preventing or treating a renal cell injury disease, which includes administering renal progenitor cells induced by the above-described method.

In yet another aspect, the present invention provides renal progenitor cells reprogrammed by the above-described method to be used in the method of preventing or treating a renal cell injury disease.

In yet another aspect, the present invention provides a pharmaceutical composition, which includes renal progenitor cells reprogrammed by the above-described method to be used in the method of preventing or treating a renal cell injury disease.

In yet another aspect, the present invention provides a use of the composition containing renal progenitor cells reprogrammed by the above-described method as an active ingredient to be used in preventing or treating a renal cell injury disease.

In yet another aspect, the present invention provides a use of renal progenitor cells reprogrammed by the above-described method for preparing a drug for preventing or treating a renal cell injury disease.

The reprogrammed renal progenitor cells of the present invention are cells having multipotency enabling differentiation into glomerular podocytes or renal tubular cells, and can restore injured or lost renal cells, thereby treating a disease generated by the injury or loss of the renal cells without limitation.

Specifically, a disease generated by the injury of renal cells may be selected from the group consisting of acute/chronic renal failure, glomerulonephritis, nephrotic syndrome, nephropyelitis, polycystic nephropathy, and an end-stage renal disease.

The pharmaceutical composition of the present invention may further introduce a drug delivery system to continuously increase its efficacy. For example, the pharmaceutical composition of the present invention may be contained in a delivery system in the form of a polymer hydrogel, a polymer micelle, an emulsion, a liposome, a polymer particle or a micro needle, and may include natural and synthetic polymers and an inorganic material as materials for constituting such a system.

The pharmaceutical composition of the present invention may further include a suitable carrier, excipient or diluent which is conventionally used in the preparation of a pharmaceutical composition of the present invention. Specifically, the pharmaceutical composition may be used in an oral formulation such as a powder, granules, a tablet, a capsule, a suspension, an emulsion, a syrup, or an aerosol, a quasi-drug, a suppository, or a sterilized injectable solution according to a conventional method. In the present invention, the carrier, excipient and diluent, which can be included in the pharmaceutical composition, may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In preparation, a diluent or excipient such as a filler, an expander, a binder, a wetting agent, a disintegrating agent or a surfactant was used. Solid preparations for oral administration include a tablet, a pill, a powder, granules and a capsule, and these solid preparations are formulated by mixing at least one excipient, for example, starch, calcium carbonate, sucrose, or gelatin. In addition, in addition to simple excipients, lubricants such as magnesium stearate and talc are also used. As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration may be a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurinum, or glycerogelatin may be used.

A content of the formulation included in the pharmaceutical composition according to one embodiment of the present invention is not particularly limited, but may be 0.0001 to 50 wt%, and more preferably 0.01 to 10 wt% based on the total weight of the final composition.

The pharmaceutical composition of the present invention may be administered at a pharmaceutically effective amount, and the term "pharmaceutically effective amount" used herein refers to an amount sufficient for treating or preventing a disease at a reasonable benefit/risk ratio applicable for medical treatment or prevention, and an effective dosage may be determined by parameters including the severity of a disease, the activity of a drug, a patient's age, weight, health condition and gender, the patient's sensitivity to a drug, the administration time, route and release rate of the composition of the present invention used herein, a treatment period, and a drug mixed or simultaneously used with the composition of the present invention, and other parameters well known in the medical field. The pharmaceutical composition of the present invention may be administered alone as a separate therapy or administered in combination with another therapy, and administered sequentially or simultaneously with a conventional therapy. In addition, the pharmaceutical composition of the present invention may be administered in a single or multiple dose(s). In consideration of all of the above-mentioned parameters, it is important to achieve the maximum effect with the minimum dose without a side effect.

The dosage of the pharmaceutical composition of the present invention may be determined by those of ordinary skill in the art in consideration of the purpose of use, the severity of a disease, a patient's age, weight and gender, the medical history, or the type of material used as an active ingredient. For example, the pharmaceutical composition of the present invention may be administered to mammals, including a human, at 10 to 100 mg/kg, more preferably, 10 to 30 mg/kg per day, and the administration frequency of the composition of the present invention may be, but is not particularly limited to, once to three times a day or several times in divided portions.

The term "prevention" used herein refers to all actions of inhibiting or delaying the occurrence of a renal cell injury disease by administration of the pharmaceutical composition according to the present invention.

The term "treatment" used herein refers to all actions involved in improving or beneficially changing a renal cell injury disease by administration of the pharmaceutical composition of the present invention.

The term "administration" used herein refers to the introduction of the pharmaceutical composition of the present invention to a subject by a suitable method, and administration may be administered in a variety of oral or parenteral routes which can reach target tissue.

The pharmaceutical composition of the present invention may be administered through any common route as long as the target tissue can be reached. The pharmaceutical composition of the present invention may be administered intraperitoneally, intravenously, intramuscularly, subcutaneously, intradermally, orally, intranasally, intrapulmonarily, or intrarectally as desired, but the present invention is not limited thereto.

### EXAMPLES

Hereinafter, the present invention will be described in more detail with reference to examples. The examples are merely provided to more fully describe the present invention, and it will be obvious to those of ordinary skill in the art that the scope of the present invention is not limited to the following examples.

### Example 1: Isolation of urine cells from urine

A method of isolating urine cells from urine was based on the technique developed by Sutherland and Bain of the UK in 1972, and in detail, it is as follows.

First, 1,000 g of urine provided from a donor was centrifuged for 10 minutes. After a supernatant was removed, the pellet remaining in the lower layer was diluted in 20 mL of a PBS solution containing an antibiotic such as 1% penicillin/ streptomycin/ amphotericin B. Afterward, 1,000 g of the diluted PBS + pellet solution was centrifuged for 10 minutes. After the removal of the supernatant, the pellet remaining in the lower layer was diluted with 1 mL of a DMEM/F12-based basal medium containing 1% penicillin/streptomycin/amphotericin B, 1% L-glutamine and 10% FBS and seeded in a gelatin-coated 12-well cell culture plate. Afterward, after culturing by adding the basal medium by 1 mL for 3 days, the resulting cells were cultured by changing the basal medium into a growth medium prepared by adding 1% penicillin/streptomycin, 1% L-glutamine, 5% FBS, 10 ng/ml bFGF and 10 g/ml EGF to a base medium in which DMEM and REGM (Lonza) were mixed at 1:1.

### Example 2: Introduction of reprogramming factor into urine cells

Vectors were prepared, using a human 293-derived retrovirus packaging cell line, 293GPG, by introducing a combination of reprogramming factors into vectors pMXs-Oct4, pMXs-Klf4, pMXs-Sox2, pMXs-cMyc, pMXs-Slug, pMXs-Sixl, pMXs-Six2, pMXs-Osrl, pMXs-Pax2 and pMXs-Eyal, which were prepared by inserting nucleotide sequences encoding Oct4, Klf4, Sox2, c-Myc, Slug, Six1, Six2, Osr1, Pax2 and Eya1 proteins into pMXs vectors, respectively. Reprogramming factor-introduced urine cells were prepared by injecting the vectors into which the combination of the reprogramming factors was introduced into the urine-derived cells isolated and cultured in Example 1 using Lipofectamine 2000 (Life Technologies).

The reprogramming factor-introduced urine cells were cultured based on a medium in which DMEM and REGM were mixed at 1:1 in a gelatin-coated 6-well cell culture plate in an environment containing 1% penicillin/streptomycin, 1% L-glutamine, 5% FBS, 10 ng/ml bFGF and 10 g/ml EGF for 2 days.

### Example 3: Induction of urine-derived reprogrammed renal progenitor cells

The reprogramming factors-introduced urine-derived cells were seeded on a Matrigel-coated cell culture plate, and then cultured in a renal progenitor cell induction and expansion medium in which 100 ng/ml of FGF9, 30 ng/ml of BMP7, 1.25 µM CHIR99021 and 10 µM Y-27632 were added to an advanced RPMI 1640 medium (Gibco) containing 1 µg/ml of heparin, 125 nM LDN-193189 and 0.5% L-glutamine for 10 to 13 days. When the induction was completed, the generation of a colony of the renal progenitor cells was able to be confirmed (FIGS. 1A and 2).

Particularly, it was confirmed that the combination (5F : Oct4, Klf4, Sox2, c-Myc, Slug) in which Slug was added to the combination of Yamanaka factors (Oct4, Klf4, Sox2 and c-Myc) has excellent self-renewal capacity and colony forming capacity (FIG. 1B). However, compared with the case in which all of five factors were introduced, male- and female-derived renal progenitor cells, which were induced from four-factor combinations from which each factor was removed one by one, showed very low colony forming capacity (FIG. 1B). In addition, even in the mRNA level analysis for SIX2, which is a representative renal progenitor cell marker gene, renal progenitor cells into which all of five factors were introduced showed much higher expression levels than when induced from four-factor combinations or embryonic stem cells (FIG. 1C).

A colony of induced reprogrammed renal progenitor cells was collected, and cultured in a Matrigel-coated cell culture plate containing the renal progenitor cell induction and expansion medium. The reprogrammed renal progenitor cells were subcultured using a 0.5 mM EDTA solution or Accutase solution, and in the following examples, various characteristics of the reprogrammed renal progenitor cells were verified.

### Example 4: Analysis of molecular biology characteristics of reprogrammed renal progenitor cells

### 4-1: Confirmation of expression of renal progenitor cell marker gene

Embryonic stem cell-derived renal progenitor cells, as a positive control, were subjected to mRNA-level analysis by RT-PCR, confirming that reprogrammed renal progenitor cells express renal progenitor cell marker genes such as SIX2, CITED1, WT1 and NCAM1 (FIGS. 3 and 4). Primer sequences used herein are as follows.
SIX2: forward-CTCAAGGCACACTACATCGAG (SEQ ID NO: 1)
   reverse-GTTGTGGCTGTTAGAATTGGA (SEQ ID NO: 2)
CITED 1: forward-CAGCATCACTTCCCGCCAATTT (SEQ ID NO: 3)
   reverse-TTGCGATCTTTCACCGCAAGG (SEQ ID NO: 4)
WT1: forward-TGTGTGCTTACCCAGGCTGCAA (SEQ ID NO: 5)
   reverse-CCGGGAGAACTTTCGCTGACAA (SEQ ID NO: 6)
NCAM1: forward-CGATCTCATGGTTTCGGGATGG (SEQ ID NO: 7)
   reverse-TCATCAAACTGCACCTGGGCTG (SEQ ID NO: 8)

### 4-2: Confirmation of pluripotent marker gene expression

According to the expression of pluripotent marker genes such as NANOG and OCT4, safety from the risk of tumorigenesis, which induced pluripotent stem cells have, was confirmed.

The mRNA-level analysis was performed on induced pluripotent stem cells as a positive control through RT-PCR to confirm the expression of pluripotent marker genes such as NANOG and OCT4 in reprogrammed renal progenitor cells (FIG. 5). Primer sequences used herein are as follows.
NANOG: forward-ATAGCAATGGTGTGACGCAG (SEQ ID NO: 9)
   reverse-GATTGTTCCAGGATTGGGTG (SEQ ID NO: 10)
OCT4: forward-GACAGGGGGAGGGGAGGAGCTAGG (SEQ ID NO: 11)
   reverse-CTTCCCTCCAACCAGTTGCCCCAAAC (SEQ ID NO: 12)

### 4-3: Confirmation of expression of renal progenitor cell marker at protein level

The protein-level expression of renal progenitor cell markers such as SIX2 and CITED 1 in reprogrammed renal progenitor cells was confirmed through immunostaining (FIG. 6). As antibodies, SIX2 (11562-1-AP, Proteintech) and CITED1 (H00004435, Abnova) were used.

In addition, western blotting showed that renal progenitor cells reprogrammed from female and male urine-derived cells express renal progenitor cell marker proteins such as SIX2 and CITED 1 (FIG. 7).

### 4-4: Karyotype analysis and quantitative analysis of mRNA of renal progenitor cell marker

To verify the molecular biology characteristics of the reprogrammed renal progenitor cells, it was confirmed, through karyotype analysis, that reprogrammed renal progenitor cells conserve normal chromosomes over time (FIG. 8).

Subsequently, through the quantitative analysis of mRNA, it was confirmed by qRT-PCR and FACS that reprogrammed renal progenitor cells maintained a certain amount of the expression of a renal progenitor cell marker gene such as SIX2 over time (FIG. 9).

### 4-5: Confirmation of renal development-related gene expression level

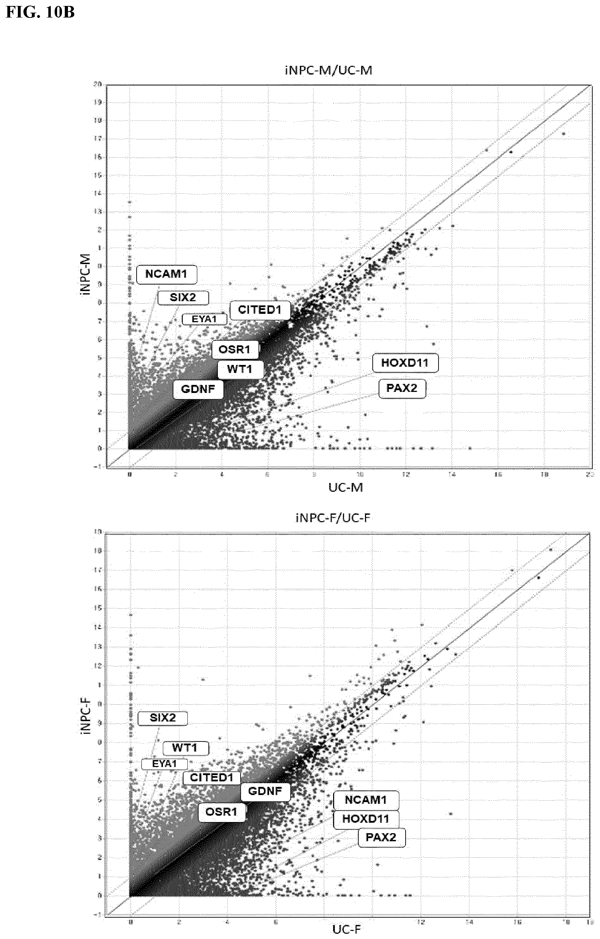

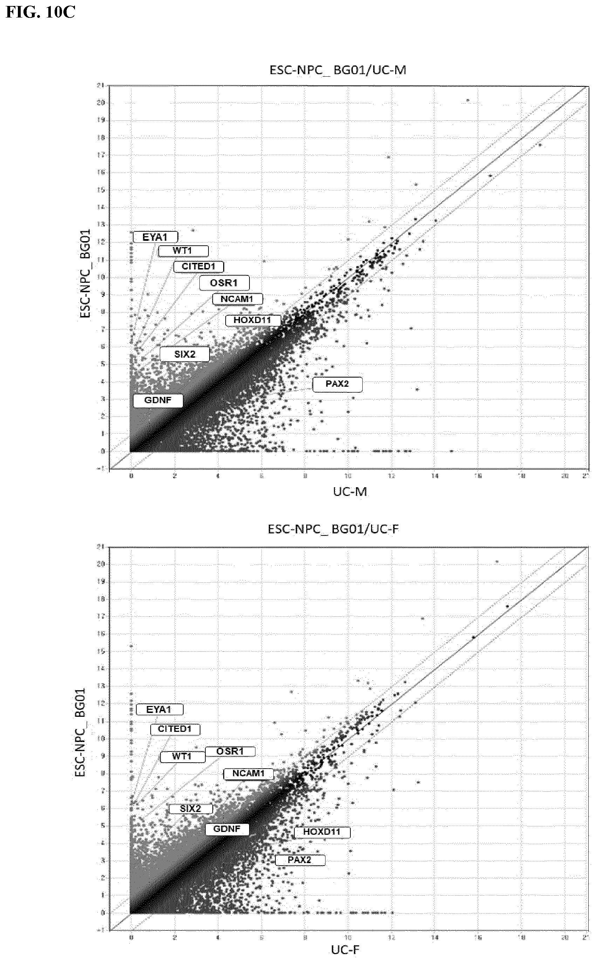

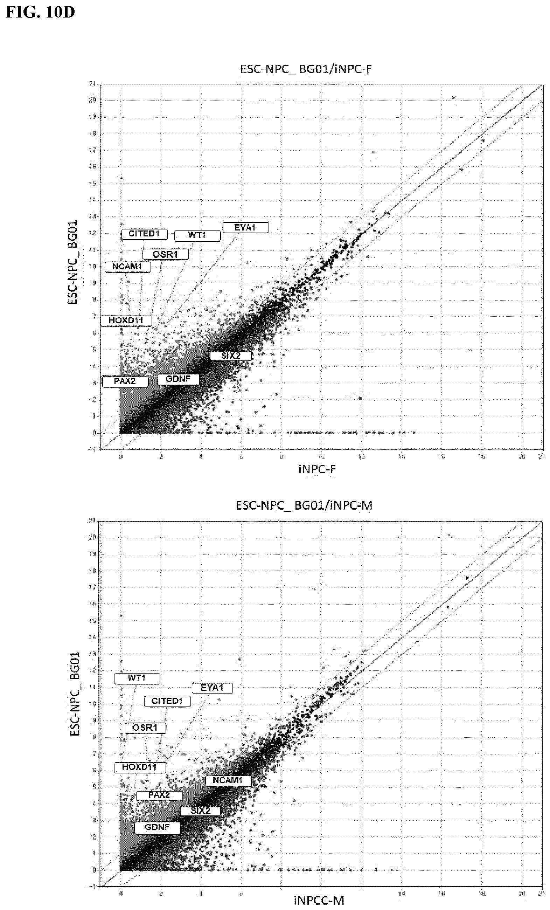

Through total RNA sequencing, it was confirmed that reprogrammed renal progenitor cells show mRNA and lnc-RNA expression patterns similar to those of renal progenitor cells derived from embryonic stem cells rather than original urine-derived cells at a renal development-related gene expression level (global gene expression level) (FIG. 10).

In addition, through total RNA sequencing, it was confirmed whether reprogrammed renal progenitor cells show mRNA and lnc-RNA expression patterns similar to those of renal progenitor cells derived from embryonic stem cells rather than original female or male urine-derived cells at an expression level of a renal development-related gene among all genes, and the commonality of renal development-related mRNA expression was confirmed through Venn diagram analysis (FIGS. 11 and 12).

Furthermore, through total RNA sequencing, genes associated with renal development among all expressed genes were divided by details, and the similarity between renal progenitor cells reprogrammed from urine cells and renal progenitor cells derived from BG01 embryonic stem cells was confirmed at a gene expression level for each group (FIG. 13).

### Example 5: Analysis of differentiation capacity of reprogrammed renal progenitor cells

### 5-1: Analysis of differentiation capacity into glomerular podocytes

To differentiate reprogrammed renal progenitor cells into glomerular podocytes, renal progenitor cells were cultured in a glomerular podocyte differentiation medium prepared by adding 1% penicillin/streptomycin, 1% L-glutamine, 10% FBS, 100 nM vitamin D3 and 60 µM all-trans retinoic acid to a DMEM/F12 medium for 7 days to induce differentiation. As a result, it was confirmed that the glomerular podocyte marker genes such as nephrin, synaptopodocin and podocalyxin are expressed (FIG. 14).

RT-PCR primer sequences used for glomerular podocyte marker gene analysis are as follows:
Nephrin: forward-TGGCTCGGACCAAACCAACATT (SEQ ID NO: 13)
   reverse-AGGGCCTCATACCTGATGCAGA (SEQ ID NO: 14)
Synaptopodocin: forward-CGCTCACCACACCAACTTCTAA (SEQ ID NO: 15)
   reverse-CTAGAAAGTGGCAGGCTCTGTG (SEQ ID NO: 16)
podocalyxin: forward-CTTGAGACACAGACACAGAG (SEQ ID NO: 17)
   reverse-CCGTATGCCGCACTTATC (SEQ ID NO: 18)

In addition, each of synaptopodocin and POXDL was stained through immunostaining, confirming that the reprogrammed renal progenitor cells can be differentiated into glomerular podocytes (FIG. 15). As antibodies, naptopodocin (SC-21537, Santa Cruz Biotechnology) and POXDL (AF1658, R&D Systems) were used.

### 5-2: Analysis of differentiation capacity into renal tubular cells

Differentiation into renal tubular cells were induced by culturing renal progenitor cells in a renal tubular cell differentiation medium in which 1% penicillin/streptomycin, 1% L-glutamine, 10% FBS,1X ITS, 20 ng/mL hEGF (human epidermal growth factor), 1 nM tri-iodothyronine and 100 ng/ml hydrocorticone were added to a DMEM/F12 medium for 21 days. As a result, it was confirmed that renal tubular cell marker genes such as CD13 and AQP1 are expressed (FIG. 16).

RT-PCR primer sequences used for the analysis of renal tubular cell marker genes are as follows.
CD13: forward-CCATGAAGGCCGAGTTCAACA (SEQ ID NO: 19)
   reverse-ATGAAGGCCAGCAAGTACGTG (SEQ ID NO: 20)
AQP1: forward-ATGCCGACGACATCAACTCCAG (SEQ ID NO: 21)
   reverse-TGAGTCGGTGAGCAACTTTGGG (SEQ ID NO: 22)

In addition, each of LTL, AQP1 and E-cadherin was stained through immunostaining, confirming that the reprogrammed renal progenitor cells can be differentiated into renal tubular cells (FIG. 17). As antibodies, LTL (B-1325, Vector Labs), AQP1 (SC32737, Santa Cruz Biotechnology) or E-cadherin (610181, BD Biosciences) were used.

### 5-3: Analysis of differentiation capacity into nephron-like tissue

Renal progenitor cells were cultured for 1 day in NPEM in which 1% penicillin/streptomycin (P/S), 1% L-glutamine (L/G), 100 ng/ml FGF9, 30 ng/ml BMP7, 1.25 µM CHIR, 125 nM LDN, 10 µM Y27632 and 10 ng/ml heparin were added to advanced RPMI 1640 (Gibco), cultured in a differentiation medium in which 1% P/S, 1% L/G, 10ng/ml FGF9 and 3 µM CHIR were added to an ARPMI medium for 2 days, cultured in a differentiation medium in which 1% P/S, 1% L/G and 10 ng/ml FGF9 were added to an ARPMI medium for 3 days, and cultured in a medium in which 1% P/S and 1% L/G were added to an ARPMI medium for 7 to 14 days, thereby inducing the differentiation into nephron-like tissue. As a result, it was confirmed that the renal progenitor cells express the glomerular podocyte marker gene PODXL and the renal tubular cell marker gene LTL through immunostaining (FIG. 18). As antibodies, PODXL (AF1658, R&D Systems) and LTL (B-1325, Vector Labs) were used.

### Industrial Applicability

Since the present invention can mass production of personalized reprogrammed renal progenitor cells using urine cells, which are somatic cells that can be easily and repeatedly obtained with inconvenience and without pain, the reprogrammed renal progenitor cells can be applied to the field of incurable diseases and production of cell therapy products, which can expand to the fields of renal injury healing and renal renewal.

As specific parts of the specification have been described in detail above, although it is clear to those skilled in the art that this specific technique is merely a preferred embodiment, the scope of the specification is not limited thereto. Thus, the substantial scope of the specification will be defined by the accompanying claims and their equivalents.

## Claims

1. A method of inducing direct reprogramming of urine cells into renal progenitor cells, comprising:
(a) culturing urine cells isolated from urine;
(b) introducing reprogramming factors such as i) a nucleic acid encoding an Oct4 protein, ii) a nucleic acid encoding an Sox2 protein, iii) a nucleic acid encoding an Klf4 protein, and iv) a nucleic acid encoding an c-Myc protein and v) a nucleic acid encoding a Slug protein into the cultured urine cells;
(c) culturing the reprogramming factor-introduced urine cells in a renal progenitor cell culture medium to induce reprogramming into renal progenitor cells; and
(d) selecting the reprogrammed renal progenitor cells having the characteristics of renal progenitor cells from the cells in which direct reprogramming into renal progenitor cells is induced.

2. The method of claim 1, wherein the urine cells are urine-derived somatic cells.

3. The method of claim 1, wherein, in Step (b), a virus vector into which the reprogramming factor is inserted is directly introduced into the urine cells.

4. The method of claim 1, wherein the culture medium in Step (c) contains FGF9, BMP7, CHIR99021 and Y-27632.

5. The method of claim 4, wherein the culture medium further contains heparin, LDN-193189 or L-glutamine.

6. The method of claim 4, wherein the cells are cultured in a Matrigel, laminin, fibronectin, gelatin or collagen-coated culture plate.

7. A pharmaceutical composition for preventing or treating a renal cell injury disease, comprising the renal progenitor cells reprogrammed by the method of claim 1 as an active ingredient.

8. The composition of claim 7, wherein the renal cell injury disease is selected from the group consisting of acute/chronic renal failure, glomerulonephritis, nephrotic syndrome, nephropyelitis, polycystic nephropathy and an end-stage renal disease.
